# EUROPÄISCHE PATENTSCHRIFT

(11) **EP 1 978 949 B1**
(45) Veröffentlichungstag und Bekanntmachung des Hinweises auf die Patenterteilung: **17.10.2012**
(21) Anmeldenummer: 07701300.1
(22) Anmeldetag: 05.02.2007
(51) Int. Cl.: A61K 31/122, A61K 31/205, A61K 31/355, A61K 31/519, A61K 33/04, A61K 33/30, A61K 38/06, A61K 45/06, A61P 15/08

(54) **KOMBINATIONSPRÄPARAT ZUR VERBESSERUNG DER SAMENQUALITÄT**
COMBINATION PREPARATION FOR IMPROVING SPERM QUALITY
PRÉPARATION COMBINÉE DESTINÉE À AMÉLIORER LA QUALITÉ DU SPERME

(30) Priorität: 03.02.2006 AT 1722006
(43) Veröffentlichungstag der Anmeldung: 15.10.2008
(73) Patentinhaber: Fapa Vital Anstalt, 9494 Schaan (LI)
(72) Erfinder: ANNERL, Brigitte, 1190 Wien (AT)
(74) Vertreter: Hasler, Erich
(86) Internationale Anmeldenummer: PCT/AT2007/000055
(87) Internationale Veröffentlichungsnummer: WO 2007/087667

(56) Entgegenhaltungen:
- EP-A- 1 714 658
- WO-A-03/032751
- WO-A-03/086080
- WO-A-2007/003007
- US-A1- 2003 224 070
- US-B1- 6 368 617
- SINCLAIR S: "MALE INFERTILITY: NUTRITIONAL AND ENVIRONMENTAL CONSIDERATIONS" ALTERNATIVE MEDICINE REVIEW, THORNE RESEARCH INC., SANDPOINT,, US, Bd. 5, Nr. 1, 2000, Seiten 28-38, XP008014042 ISSN: 1089-5159

## Beschreibung

Die Erfindung betrifft ein Kombinationspräparat gemäß Anspruch 1 sowie ein Verfahren zur Herstellung eines Kombinationspräparats zur Behandlung der eingeschränkten Fruchtbarkeit bei Männern gemäß Anspruch 11.

Die Fertilitätsbehandlung bei Paaren mit unerfülltem Kinderwunsch nimmt in der praktischen Medizin in den letzen Jahren einen immer größeren Stellenwert ein. Unfruchtbarkeit betrifft mittlerweile schon eines von sechs Kinderwunschpaaren. Die Ursache für einen unerfüllten Kinderwunsch kann sowohl bei der Frau als auch beim Mann liegen, wobei der Faktor Mann in den letzten Jahren zunehmend an Bedeutung gewonnen hat. Im Gegensatz zur Frau, bei der eine Reihe von therapeutischen Möglichkeiten zur Verfügung steht, sind die Optionen für eine Verbesserung des einzigen und entscheidenden Faktors, nämlich der Samenqualität, beim Mann sehr begrenzt.

Sowohl die Qualität als auch die Quantität des männlichen Samens werden durch zahlreiche Faktoren beeinflusst. Diverse Umweltbelastungen (Schadstoffe, Schwermetalle etc.) belastende Lifestylefaktoren (wie z.B. Nikotin, Alkohol etc.) und veränderte Lebensumstände (ausgeprägter Stress) belasten heute die Samenentwicklung sowie die Beweglichkeit, das Aussehen und die Anzahl der Spermien. Die Samenproduktion ist ein sehr komplizierter und biologisch aufwändiger Vorgang, der über mehrere Wochen andauert und der leicht negativ beeinflussbar ist. Im Rahmen der Samenzellreifung benötigt der Körper bei den unterschiedlichen Entwicklungsschritten spezielle Hilfsstoffe. Zu diesen zählen verschiedene Aminosäuren, Spurenelemente, Vitamine und vitaminähnliche Substanzen. Nur mit diesen speziellen Vitalstoffen ist die optimale Voraussetzung für eine normale und gesunde Spermienentstehung gegeben. Durch gewisse Lebensumstände und einen falschen Lebensstil kann ein Mangel an diesen Substanzen rasch eintreten und wird von den Betroffenen meist nicht bemerkt. Aus diesem Grund ist eine regelmäßige Zufuhr dieser Stoffe in ausreichender Menge unabdingbar.

Viele einzelne, für den Verlauf der Samenzellreifung wichtige Substanzen wurden bereits wissenschaftlich untersucht und beschrieben. Die Wirksamkeit jedes einzelnen Vitalstoffes ist hinreichend bekannt.

Die Einzelkomponenten und deren Wirkungsweisen sind alle für sich in separater Weise vorliegend bekannt. Im folgenden wird ein Überblick über die einzelnen Wirkstoffe gegeben:

L-Carnitin [6645-46-1]: L-Carnitin ist eine körpereigene Substanz, die den Samenzellen als Energiesubstrat dient. L-Carnitin kann eine Verbesserung der Spermienbeweglichkeit und der Spermienanzähl bewirken.

L-Arginin [74-79-3]: L-Arginin ist eine. Aminosäure, die vom Körper in großen Mengen benötigt wird und in Untersuchungen zu einer signifikanten Verbesserung der Spermienanzahl und der Spermienbeweglichkeit geführt hat.

Coenzym Q₁₀ [303-98-0]: Das Ubichinon Coenzym Q₁₀ kann zu einer Verbesserung der Befruchtungsrate, einer Zunahme der Samenzellanzahl und einer Verbesserung der Spermienbeweglichkeit führen.

Vitamin E, insbesondere α-Tocopherol [59-02-9]: Vitamin E kann die Spermienbeweglichkeit verbessern und fördert die Fähigkeit der Samenzelle, sich mit der Eizelle zu vereinen.

Zink: Zink ist ein lebensnotwendiges Spurenelement und wirkt einerseits als Antioxidans, d.h. als Substanz, die freie Radikale bindet oder die Neubildung verhindert, weiters bewirkt Zink in der Beteiligung an zahlreichen biochemischen Prozessen im Körper eine Verbesserung des Samenzelldichte, eine Zunahme der schnell beweglichen Spermien und eine Erhöhung des für die Samenzellreifung wichtigen Testosteronspiegels.

Folsäure [75708-92-8]: Folsäure ist unentbehrlich für das Zellwachstum, die Zellteilung (Blutbildung) sowie für den Nervenstoffwechsel und ist am Schutz des Herz-Kreislaufsystems beteiligt. Schon lange ist die positive Wirkung dieses Vitamins auf die Samenqualität bekannt, ausführlich untersucht und wird zur Unterstützung empfohlen.

Glutathion [70-18-8] und Selen: Glutathion und Selen sind hochwirksame Radikalfänger und können die Beweglichkeit der Samenzellen deutlich verbessern.

Es sind aus dem Stand der Technik auch einige Präparate zur Verbesserung der Samenqualität des Mannes bekannt, bei denen einige dieser Wirkstoffe miteinander kombiniert werden.

So wird beispielsweise in der WO 02/003974 ein Präparat zur Verbesserung der Samenqualität beschrieben, das auf der Kombination von Folsäure mit Zinksulfat beruht.

Weiters ist in der US 6,338,862 ein Präparat zur Behebung von erektilen Dysfunktionen beschrieben, das L-Arginin, Ginseng und verschiedene Vitamine der B-Gruppe miteinander kombiniert.

Bislang ist es jedoch nach wie vor äußerst schwierig und nach vielfacher Meinung unmöglich, durch medikamentöse Behandlung eine signifikante Verbesserung der Fertilität zu erreichen auch wenn dies oft propagiert und behauptet wird.

**Aufgabe** der Erfindung ist es daher ein wirkungsvolles Präparat unter anderem zur Verbesserung der Samenqualität des Mannes zu schaffen.

Diese Aufgabe wird durch die Merkmale des Anspruchs 1 gelöst.

Die im Kombinationspräparat vereinten und sorgfältig ausgesuchten Einzelsubstanzen haben sich in ihrer Wirksamkeit zur Verbesserung der Samenqualität bewährt und sind bereits erprobt.

In Studien zeigte sich die überraschende Wirkung des neuen Kombinationspräparates, die die Summe der zu erwartenden Einzelwirkungen weit übersteigt und auch die Wirksamkeit aller bisher verfügbaren Zusammensetzungen weit übertrifft. Durch die Verabreichung des erfindungsgemäßen Kombinationspräparats konnte eine eindeutige Verbesserung der Samenqualität bis hin zu einem völlig normalen Spermiogramm erzielt werden. Derartige Behandlungserfolge sind bis dato nicht bekannt und auch die überraschende Höhe der Wirksamkeit ist äußerst vorteilhaft für die betroffenen Patienten. Alle fruchtbarkeitsrelevanten Parameter des Spermiogramms wie z.B. Dichte, Motilität und Morphologie der Spermien wurden signifikant erhöht.

Wie die Literatur beweist sowie durch Vergleichsversuche bestätigt wurde, konnte keine der Einzelsubstanzen vergleichbare Ergebnisse erzielen, was verständlich macht, dass nur die spezielle Kombination der verwendeten Substanzen zum gewünschten Erfolg führt.

In diesem Zusammenhang ist besonders auf die vorteilhafte Wirkung von Arginin und die durch Arginin bewirkten synergistischen Effekte hinzuweisen, wie später detailliert ausgeführt wird.

In den abhängigen Ansprüchen werden vorteilhafte Weiterbildungen der Erfindung dargestellt.

Die vorteilhaften Merkmale des Anspruchs 2 beschreiben eine besonders wirksame Zusammensetzung des Kombinationspräparates. Durch eine derartige Zusammensetzung werden die Zusammenwirkungen der Einzelsubstanzen untereinander optimiert und die Synergieeffekte zusätzlich verstärkt.

Die Merkmale des Anspruchs 3 verstärken die Wirkung zusätzlich, wobei es vorteilhaft ist, wenn die Konzentrationen des Anspruchs 4 eingehalten werden. Aufgrund der hohen antioxidativen Fähigkeiten und der bekannten großen Rolle der freien Radikale im Rahmen der Samenzellreifung ist die Verabreichung der alpha-Liponsäure bei schlechtem Spermiogramm sehr vorteilhaft. Dies wurde in einer Anwendungsbeobachtung ebenfalls bereits klinisch erfolgreich getestet. Außerdem ist bekannt, dass die alpha-Liponsäure den so wichtigen Glutathionspiegel hebt.

Die Merkmale des Anspruchs 5 bewirken einen weiteren wirkungssteigernde Effekt, wobei es vorteilhaft ist, wenn die Konzentrationen des Anspruchs 6 eingehalten werden. N-Acetylcystein (NAC) ist eine für die ausreichende Glutathionproduktion sehr wichtige Substanz. Das vorteilhafte und überraschend starke synergistische Zusammenspiel zwischen Glutathion, NAC und alpha-Liponsäure trägt zur weiteren Verbesserung der Samenqualität bei.

Sowohl im Tierversuch als auch beim Menschen erweist sich die Gabe von Vitamin C gemäß den Merkmalen der Ansprüche 7 und 8, als äußerst nützlich zur Verbesserung der Samenqualität. Vitamin C reduziert signifikant die für die Samenproduktion schädliche Lipidoxidation in den Hoden und führt so zu einer Verbesserung der Samenqualität im Sinne einer Erhöhung der Samenzelldichte und einer Steigerung des Testosteronspiegels.

Die Wirkung des Kombinationspräparates kann durch die Vorsehung von weiteren Merkmalen gemäß Anspruch 9 noch weiter gesteigert werden.

In einer Studie wurden 66 Patienten mit schlechter Samenqualität untersucht. Durch die Gabe von Ginseng Extrakt kam es zu einer Erhöhung der Samenzellkonzentration, der Beweglichkeit und einer Erhöhung des Testosteronspiegels. Der Zusatz von Ginseng erbrachte auch weitere kombinatorische Effekte, insbesondere betreffend die Motilität der Samenzellen.

Sowohl im Tierversuch als auch beim Menschen erwies sich die Gabe von Vitamin B12 als ausgezeichnete Möglichkeit sowohl die Samenzellkonzentration als auch die Motilität entscheidend zu verbessern.

Die Abhängigkeit der Spermiogenese vom Thiamin ist eine neue Erkenntnis. Im Tierversuch entdeckte man, dass Versuchsobjekte mit einem Thiaminmangel im Vergleich zur Kontrollgruppe ohne Mangel Defizite in der Spermiogenese aufweisen und dadurch sehr eingeschränkt fertil sind. Zusätzlich vermutet man eine negative Beeinflussung des Thiaminmangels auf den Hormonhaushalt. Auch die Zugabe von Thiamin zeigte überraschende Erhöhungen der Wirksamkeit gegenüber der bekannten Einzelsubstanz. Selbiges gilt auch für Biotin.

Ein besonders vorteilhaftes Beispiel eines hochwirksamen Kombinationspräparates, dessen Wirksamkeit auch durch klinische Untersuchungen nachweislich bestätigt wurde, ist in den Merkmalen des Anspruchs 10 beschrieben. Dieses Präparat besteht aus den genannten Wirkstoffen, wobei sich bereits aus der relativ geringen Anzahl der kombinierten Wirkstoffe in vorteilhafter Weise die gewünschten überraschenden Effekte einstellen.

Weiters ist es Aufgabe der Erfindung, eine vorteilhafte Verwendung des Kombinationspräparates zur Herstellung eines Arzneimittels vorzusehen.

Diese Aufgabe wird durch die Merkmale des Anspruchs 11 gelöst, in dem das Kombinationspräparat als wirksames Arzneimittel zur Verbesserung der Samenqualität bzw. zur Behandlung von eingeschränkter Fruchtbarkeit eingesetzt wird. Außerdem kann das Kombinationspräparat zur Behandlung von Subfertilität eines männlichen Individuums, insbesondere eines Menschen, zur Behandlung bzw. Optimierung einer grenzwertigen Normozoospermie, zur Behandlung einer Asthenozoospermie, Oligozoospermie, Teratozoospermie, Oligo-Astheno-Terato-Zoospermie, insbesondere OAT I, OAT II und/oder OAT III, Kryptozoospermie, Parvisemie und/oder zur Verbesserung einer beeinträchtigten Kapazitationsfähigkeit verwendet werden.

In diesem Zusammenhang ist es besonders vorteilhaft, die Merkmale des Anspruchs 12 vorzusehen, wodurch die Wirksamkeit erhöht wird.

Durch die Merkmale des Anspruchs 13 wird eine wirksame Dosierung vorgegeben, die einerseits für den Körper verträglich ist und andererseits die oben wirksamen Effekte aufweist.

Unfruchtbarkeit betrifft mittlerweile schon eines von sechs Kinderwunschpaaren. Mittlerweile beginnt man die Pathologien sowohl auf biochemischer als auch auf zellulärer Ebene, die dann in weiterer Folge die Produktion defekter Samenzellen verursachen, zu verstehen.

ROS (reactive oxygen species) sind Reaktionsprodukte, die die Lipide der Samenzellmembran im Plasma angreifen und dadurch eine Peroxidationskaskade initiieren. Dadurch verlieren die Spermatozoen die Fähigkeit sich zu bewegen, die Akrosomenreaktion und die Fusion zwischen Samenzelle und Eizelle ist nicht mehr möglich. ROS behindern die Mitochondrienfunktion und sie beeinflussen die Synthese der DNA, der RNA und die Bildung von Proteinen negativ. An dieser Stelle kommen bereits zwei Substanzen des Kombinationspräparats zum Einsatz.

L-Carnitin und Coenzym Q₁₀ wirken auf zellulärer Ebene, indem sie entscheidend am Energiestoffwechsel der Mitochondrien in den Zellen beteiligt sind.

L-Arginin, eine bedeutende Aminosäure, stellt den Gründbaustein für die proteinbiosynthese dar. Nur durch einen ausreichenden Vorrat dieser Substanzen im Körper werden die oben genannten Funktionen überhaupt gewährleistet.

Arginin bewirkt im erfindungsgemäßen Kombinationspräparat einen spermienqualitätssteigernden und fruchtbarkeitsfördernden Effekt auf drei Arten. Es bewirkt primär einen selbständigen positiven Effekt auf die Spermienmotilität und Anzahl der Spermien. Zweitens bewirkt der außergewöhnlich starke Effekt von Arginin auf die Regulation von Stickstoffmonoxid eine Optimierung des Stoffwechselmilieus als Ausgangsituation für einen verbesserten Effekt aller anderen Inhaltsstoffe. Arginin hat somit einen vorteilhaften Einfluss auf die Wirksamkeit der weiteren Bestandteile des Kombinationspräparats. Die potente Reduktion des oxidativen Stresses verschafft den funktionell wirksamen Bestandteilen des Präparates eine wesentlich verbesserte Ausgangssituation und verstärkt damit deutlich deren Effekt auf Beweglichkeit und Motilität. Dies ist eine Begründung, wieso die Kombination der Einzelsubstanzen eine Verbesserung des Einflusses auf Motilität und Spermienzahl bringt, welche über den Effekt der Einzelsubstanzen hinausgeht. Wie in der zitierten eigenen Untersuchung gezeigt werden konnte, verbessert sich der Effekt überraschenderweise derart, dass bei Studienende neun normale Spermiogramme (29%) erhalten wurden. Der dritte Wirkungsmechanismus von Arginin beruht auf der deutlichen Verbesserung der Kapazitationsfähigkeit der Spermien, nämlich der Vorbereitung des und des Eindringens selbst des Spermiums in die Eizelle.

Diese Eigenschaft von L-Arginin begründet in Kombination mit den anderen Bestandteilen, insbesondere mit Vitamin E, Zink und Glutathion, auch die überraschende Entwicklung, dass bereits innerhalb der Studie vier Schwangerschaften und knapp nach Ende der Studie sechs Schwangerschaften beobachtet werden konnten.

Glutathion spielt eine signifikante Rolle in der antioxidativen Abwehr der Samenzellepithelien, beim Nebenhoden und allgemein im Ejakulat. Dieses hoch effektive Antioxidans kann nur in Kombination mit Selen besonders gut die Funktionalität der Samenzellen aufrechterhalten. Sowohl Hoden als auch Nebenhoden und damit die Spermatogenese sind auf die Abwehrmechanismen der Kombination von Glutathion und Selen angewiesen. Dies geschieht unter anderem über die Enzyme Superoxid Dismutase (SOD) und Glutathion Peroxidase, die eine schädigende Lipidoxidation verhindern können. Durch das Enzym Glutathion-S-Transferase werden andere fremde und belastende Substanzen (Umweltgifte) deaktiviert.

Diese beschriebenen Schutzmechanismen funktionieren nur unter bestimmten Bedingungen, in begrenztem Ausmaß und auch nur in Kombination und mit Unterstützung anderer Antioxidativa.

Dazu zählt Zink, das an fast allen wichtigen biochemischen Prozessen des Körpers beteiligt ist.

Zusätzlich bedarf es der Kombination der antioxidativ wirksamen Substanzen Vitamin E, ergänzend mit Folsäure aus dem Vitamin B Komplex. Diese beiden Vitamine spielen eine tragende Rolle bei der Spermiogenese und sind in ihrer Kombination unentbehrlich.

Somit besteht die überraschende Wirksamkeit dieses Präparates in der synergistischen Zusammenwirkung der Einzelsubstanzen.

In untenstehenden Tabellen ist beispielhaft ein erfindungsgemäßes Kombinationspräparat beschrieben, das die vorteilhaften Wirkungen entfaltet.

**Tabelle 1**

| **Inhaltsstoffe** | **Menge der aktiven Wirkstoffe pro Kapsel** | **Gew% bezogen auf die Gesamtmenge der aktiven Wirkstoffe (Beispiel)** | **empfohlene Menge [mg] pro Tag (Bereich)** | **empfohlene Menge [mg] pro Tag (Beispiel)** |
|---|---|---|---|---|
| L-Carnitin | 220 mg | 46,5 | 100 ― 5000 | 440 |
| L-Arginin | 125 mg | 26,4 | 100 ― 5000 | 250 |
| Coenzym Q₁₀ | 7,5 mg | 1,59 | 5 ― 250 | 15 |
| Vitamin E | 60 mg | 12,7 | 50 ― 1000 | 120 |
| Zink | 20 mg | 4,23 | 5 ― 100 | 40 |
| Folsäure | 0,4 mg | 0,085 | 0,1 ― 1 | 0,8 |
| Glutathion | 40 mg | 8,46 | 10 ― 1000 | 80 |
| Selen | 0,03 mg | 0,0063 | 0,01 - 1 | 0,06 |
| **aktive Wirkstoffe gesamt** | **472,93 mg** | **100,00 %** | | |

**Tabelle 2**

| **Inhaltsstoffe** | **pro Tag aufgenomme Mengen an aktiven Wirkstoffen** | **Gew% bezogen auf das Gesamtgewicht des Kombinationspräparats inkl. Füllstoffe (Beispiel) pro Kapsel** |
|---|---|---|
| L-Carnitin | 440 mg | 25,9 |
| L-Arginin | 250 mg | 14,7 |
| Coenzym Q₁₀ | 15 mg | 0,9 |
| Vitamine E | 120 mg | 7,1 |
| Zink | 40 mg | 2,4 |
| Folsäure | 800 µg | 0,0471 |
| Glutathion | 80 mg | 4,7 |
| Selen | 60 µg | 0,0035 |
| **Summe der aktiven Wirkstoffe** | | **55,7506** |
| Rest Füllstoffe (nicht aktiv wirksam) | | ca. 44,2 |
| **Summe Gesamt** | | 100,0000 |

In Tabelle 1 ist die Zusammensetzung eines besonders wirkungsvollen und klinisch in seiner Wirksamkeit bestätigten Kombinationspräparates detailliert beschreiben. Das Kombinationspräparat liegt in Kapselform vor, wobei im Inneren der Kapsel die aktiven wasserlöslichen und fettlöslichen Wirkstoffe als Granulat vorliegen.

In einer Kapsel sind somit gemäß diesem Beispiel 472,93 mg an aktiven Wirkstoffen enthalten. Gemäß Tabelle 2 entspricht dies einer Menge von ca. 56 Gew% bezogen auf das Gesamtgewicht des Kapselinhaltes. Die restlichen 44 % sind Füllstoffe, beispielsweise Stärke, die zur technischen Verarbeitbarkeit des Granulates vorteilhaft sind. Diese technischen Füllstoffe haben jedoch keine Wirksamkeit und keine positiven Effekte auf die Spermienqualität und sind dementsprechend keine aktiven Wirkstoffe im Sinne der oben genannten.

In Tabelle 1 wird weiters die prozentuelle Zusammensetzung bzw. das Verhältnis der Wirkstoffe zueinander in Gew% beschrieben. Glutathion hat hier entscheidenden Einfluss auf die Wirksamkeit des Präparates, da durch das Glutathion die kombinatorischen bzw. synergistischen Effekte verstärkt und zusätzlich angekurbelt werden.

In Tabelle 1 sind weiters in den letzten beiden Spalten die Bereiche der empfohlenen Tagesdosen angegeben sowie die empfohlene Dosierung bei Einnahme von zwei Kapseln pro Tag.

Die erfindungsgemäßen Kombinationspräparate können insbesondere Zubereitungen und/oder Zusammensetzungen sein, die für eine orale Verabreichung an ein männliches Individuum einer Säugerart gerichtet und/oder geeignet sind, umfassend aber nicht beschränkt auf pharmazeutische, diätetische oder Veterinärzubereitungen oder Zusammensetzungen und/oder Nahrungszusätze. Das Kombinationspräparat ist somit sowohl für menschliche Nahrung als auch für tierische Nahrung geeignet.

Der Begriff "eine Quelle für" bezieht sich auf eine Verbindung, die für die Verabreichung und insbesondere für die orale Verabreichung an ein männliches Individuum einer Säugerart geeignet ist, und die bei derartigen Verabreichungen dem jeweiligen Individuum die gewünschte Substanz zur Verfügung stellt und/oder biologisch zugänglich macht.

So ist unter "einer Quelle für ein Vitamin B, insbesondere Folsäure" eine Substanz zu verstehen, die zu erhöhten Spiegeln von Folsäure im Körper oder in wenigstens einem Teil davon führt, umfassend aber nicht beschränkt auf ein biologisches Fluid wie Blut, Plasma und/oder Samenflüssigkeit. Quellen von Folsäure sind beispielsweise Folsäure und deren Salze, Folatverbindungen, oder wahlweise geeignete Analoga, Vorläufer und/oder Metaboliten von Folsäure oder Folatverbindungen oder jede geeignete Kombination davon. Die Quelle(n) von Folsäure können in geeigneter reduzierter oder oxidierter Form vorliegen, wie es auch dem Durchschnittsfachmann klar ist. Einige spezifische Beispiele von geeigneten Quellen an Folsäure umfassen Mono- oder Polyglutamatformen von Folsäure, Salzen und/oder Estern von Folsäure und/oder methylierten Derivaten von Folsäure, beispielsweise Folinsäure oder 5-Methyltetrahydrofolsäure oder jede geeignete Kombination davon. Auch die reduzierte Monoglutamatform ist einsetzbar.

Der Betriff "eine Quelle für Zink" bezieht sich auf eine Verbindung, die für die Verabreichung und insbesondere für eine orale Verabreichung an ein männliches Individuum einer Säugerart geeignet ist und die nach Verabreichung diesem Individuum Zink zur Verfügung stellt und/oder biologisch verfügbar macht, insbesondere in Form von Zn²+-Ionen. Geeignete Beispiele derartiger biologisch verfügbarer Quellen von Zink umfassen biologisch verfügbare Zinksalze mit anorganischen Anionen wie Chlorid, Carbonat und Sulfat, aber ebenso Zinksalze mit organischen Anionen, wie Lactat, Gluconat, Fructosephosphate, Orotate, Citrate, Malate, Pyruvate, etc. und Zinkkomplexen mit einem organischen Molekül wie ein Aminosäure, oder eine zwei- oder dreizähnige Verbindung oder jede geeignete Kombination davon.

Selbiges gilt für die weiteren Substanzen, die ebenfalls in jeder Form, sei es als organisches oder anorganisches Salz vorliegen können, die dem Körper den aktiven Wirkstoff bioverfügbar zur Disposition stellt.

Unter dem Begriff "in wirksamen Mengen" sind solche Mengen zu verstehen, die bewusst und gewollt und in therapeutisch ausreichenden Mengen enthalten sind. Geringfügige Verunreinigungen, insbesondere solche ohne therapeutischen Effekt, sind davon nicht umfasst.

Zur Belegung der vorteilhaften und überraschenden Wirkungen wurde eine Studie durchgeführt. In dieser Studie wurden insgesamt 40 Männer mit langjährigem Kinderwunsch und mindestens 2 pathologischen Spermiogrammen in der Anamnese inkludiert. Diese Patienten erhielten das erfindungsgemäße Kombinationspräparat über einen Zeitraum von insgesamt 3 Monaten. Die tägliche Dosis waren 2 Kapseln, was einer Menge an aktiven Wirkstoffen, wie aus der letzten Spalte der Tabelle 1 ersichtlich, entspricht. Nach einer Einnahmedauer von 3 Monaten wurde ein Kontrollspermiogramm durchgeführt. Bisher konnten 18 Patienten ausgewertet werden.

Die Studie erbrachte folgende eindeutige Ergebnisse:

Das Ejakulationsvolumen, bezogen auf das Gesamtkollektiv, hat im Durchschnitt von 53,1 ml auf 66,5 ml zugenommen. Das entspricht einer Zunahme von insgesamt 13,4 ml und einer durchschnittlichen Zunahme des Ejakulationsvolumens von 0,74 ml pro Patient. Eine Zunahme wurde bei 14 von 18 Patienten festgestellt.

Die Spermiendichte nahm insgesamt von 60,7 Millionen/ml auf 94,5 Millionen /ml zu. Das entspricht einer Differenz von 33,8 Millionen /ml. Ein Anstieg der Spermienzahl konnte bei 15 von 18 Patienten verzeichnet werden. Im Durchschnitt hat also bei jedem Patienten die Spermienanzahl pro Milliliter um 18,8 Millionen zugenommen. In mehreren Einzelfällen kam es zu einer Zunahme der Spermienanzahl von bis zu 620%.

Durch die Einnahme des Kombinationspräparats nahm die Anzahl bedeutsamen, schnell beweglichen Samenzellen von insgesamt 17,1% auf 34,5%. Diese konnte somit mehr als verdoppelt werden. Auch hier trat diese Verbesserung in 15 von 18 Fällen auf. Die Gesamtbeweglichkeit, also schnell und langsam bewegliche, nahm von insgesamt 62,2% auf 88,7% zu. Dies entspricht einer Zunahme an beweglichen Samenzellen von knapp 15% pro Proband.

Die Anzahl der morphologisch unauffälligen Samenzellen konnte, bezogen auf das Gesamtkollektiv, mehr als verdoppelt werden. Die Zunahme betrug 103%.

Bei fast allen bis jetzt ausgewerteten Studienteilnehmern kam es zu einer Verbesserung aller relevanten Parameter. Bei 7 von 18 haben sich die seit Jahren bestehenden Pathologien im Spermiogramm soweit normalisiert, das die Diagnose nun Normozoospermie lautete. Bis dato sind auch 3 Schwangerschaften innerhalb der Studie entstanden.

Das Kombinationspräparat sollte zweimal täglich in Form je einer Kapsel mit etwas Flüssigkeit vor oder zu einer Mahlzeit eingenommen werden. Die Mindesteinnahmedauer beträgt 3 Monate, um jedes Stadium der Spermienentstehung optimieren zu können. Das Kombinationspräparat kann und sollte allerdings bis zum Eintreten der Schwangerschaft weiterhin eingenommen werden.

Das Kombinationspräparat liegt als verkapseltes Granulat vor, wobei das Kapselmaterial aus rein pflanzlichen Stoffen, insbesondere Cellulose, hergestellt ist. Somit ist das Präparat auch besonders für Vegetarier geeignet.

In Tabelle 3 werden weitere optional einsetzbare Wirkstoffe beschrieben, die die Wirkungen zusätzlich synergistisch verstärken, wenn zumindest einer davon zusätzlich eingesetzt wird.

**Tabelle 3**

| **weitere mögliche Inhaltsstoffe** | **empfohlene Menge [mg] pro Tag (Bereich)** | **empfohlene Menge [mg] pro Tag (Beispiel)** |
|---|---|---|
| Vitamin C (Ascorbinsäure) | 100 ― 1000 | 500 |
| Vitamin B₁₂ (Metylcobalamin) | 0,1-5 | 1,25 |
| Vitamin B₁ (Thiamin) | 10 ― 500 | 100 |
| Vitamin B₇ (Biotin) | 0,5 - 10 | 2,5 |
| Ginseng | 500 ― 2000 | 1000 |
| α-Liponsäure | 100 ― 1000 | 600 |
| N-Acetylcystein | 100 ―1000 | 600 |

## Patentansprüche

1. Kombinationspräparat zur Verbesserung der Samenqualität eines männlichen Individuums umfassend als aktive Wirksubstanzen in wirksamen Mengen:
- L-Carnitin,
- Coenzym Q₁₀,
- zumindest ein Vitamin E, insbesondere α-Tocopherol,
- zumindest eine Quelle für Zink, insbesondere Zinksulfat oder Zinkchlorid,
- zumindest eine Quelle für ein Vitamin B, insbesondere Folsäure,
- zumindest eine Quelle für Selen,
- Glutathion und
- L-Arginin
bzw. Salze davon.

2. Kombinationspräparat nach Anspruch 1, umfassend
- 25 bis 65 Gew%, insbesondere 35 bis 60 Gew%, vorzugsweise 42 bis 55 Gew%, L-Carnitin
- 0,5 bis 11 Gew%, insbesondere 1 bis 6 Gew%, vorzugsweise 1,5 bis 4 Gew%, Coenzym Q₁₀
- 4 bis 30 Gew%, insbesondere 7 bis 25 Gew%, vorzugsweise 10 bis 16 Gew%, eines Vitamin E, insbesondere α-Tocopherol,
- 0,5 bis 20 Gew%, insbesondere 1 bis 15 Gew%, vorzugsweise 2 bis 7 Gew%, einer Quelle für Zink
- 0,01 bis 1 Gew%, insbesondere 0,05 bis 0,3 Gew%, vorzugsweise 0,07 bis 0,15 Gew%, eines Vitamin B, insbesondere Folsäure,
- 0,001 bis 0,1 Gew%, insbesondere 0,002 bis 0,03 Gew%, vorzugsweise 0,005 bis 0,015 Gew%, einer Quelle für Selen
- 1 bis 25 Gew%, insbesondere 3 bis 17 Gew%, vorzugsweise 6 bis 13 Gew%, Glutathion
- 15 bis 45 Gew%, insbesondere 20 bis 35 Gew%, vorzugsweise 22 bis 29 Gew%, L-Arginin
bzw. Salze davon, wobei sich die Prozentangaben auf das Gesamtgewicht aller aktiven Wirksubstanzen beziehen.

3. Kombinationspräparat nach Anspruch 1 oder 2, **dadurch gekennzeichnet, dass** zusätzlich eine wirksame Menge von Liponsäure, insbesondere von α-Liponsäure, bzw. Salze davon, enthalten ist.

4. Kombinationspräparat nach Anspruch 3, **dadurch gekennzeichnet, dass** Liponsäure in einer Menge von 1 bis 20 Gew%, insbesondere 3 bis 17 Gew%, vorzugsweise 5 bis 10 Gew%, bezogen auf das Gesamtgewicht aller aktiven Wirksubstanzen enthalten ist.

5. Kombinationspräparat nach einem der Ansprüche 1 bis 4, **dadurch gekennzeichnet, dass** zusätzlich eine wirksame Menge von N-Acetylcystein (NAC) bzw. Salze davon, enthalten ist.

6. Kombinationspräparat nach Anspruch 5, **dadurch gekennzeichnet, dass** N-Acetylcystein (NAC) in einer Menge von 1 bis 20 Gew%, insbesondere 3 bis 17 Gew%, vorzugsweise 5 bis 10 Gew%, bezogen auf das Gesamtgewicht aller aktiven Wirksubstanzen enthalten ist.

7. Kombinationspräparat nach einem der Ansprüche 1 bis 6, **dadurch gekennzeichnet, dass** zusätzlich eine wirksame Menge mindestens eines weiteren Vitamins, insbesondere von Ascorbinsäure, bzw. Salze davon, enthalten ist.

8. Kombinationspräparat nach Anspruch 7, **dadurch gekennzeichnet, dass** Ascorbinsäure in einer Menge von 4 bis 30 Gew%, insbesondere 7 bis 25 Gew%, vorzugsweise 10 bis 16 Gew%, bezogen auf das Gesamtgewicht aller aktiven Wirksubstanzen enthalten ist.

9. Kombinationspräparat nach einem der Ansprüche 1 bis 8, **dadurch gekennzeichnet, dass** zumindest ein weiterer aktiver Wirkstoff, ausgewählt aus der Gruppe von Ginseng, Vitamin B12 bzw. Methylcobalamin, Thiamin und/oder Biotin, in wirksamen Mengen enthalten ist.

10. Kombinationspräparat nach einem der Ansprüche 1 bis 9, bestehend aus
- ca. 46 - 47 Gew% L-Carnitin
- ca. 1,5 - 1,6 Gew% Coenzym Q₁₀
- ca. 12 - 13 Gew% eines Vitamin E, insbesondere α-Tocopherol,
- ca. 4 - 5 Gew% einer Quelle für Zink
- ca. 0,08 - 0,09 Gew% eines Vitamin B, insbesondere Folsäure,
- ca. 0,006 - 0,007 Gew% einer Quelle für Selen
- ca. 8 - 9 Gew% Glutathion
- ca. 26 - 27 Gew% L-Arginin
wobei sich die Prozentangaben auf das Gesamtgewicht aller aktiven Wirksubstanzen beziehen.

11. Verwendung von L-Carnitin, Coenzym Q₁₀, zumindest einem Vitamin E, insbesondere α-Tocopherol, zumindest einer Quelle für Zink, insbesondere Zinksulfat oder Zinkchlorid, zumindest einem Vitamin B, insbesondere Folsäure, zumindest einer Quelle für Selen, Glutathion, Arginin sowie gegebenenfalls Liponsäure, Acetylcystein bzw. weiteren Wirkstoffen nach einem der Ansprüche 1 bis 10 bzw. Salze davon, zur Herstellung eines Kombinationspräparates gemäß einem der Ansprüche 1 bis 10 zur Behandlung eingeschränkter Fruchtbarkeit bzw. Subfertilität eines männlichen Individuums, insbesondere eines Menschen, zur Behandlung bzw. Optimierung einer grenzwertigen Normozoospermie, zur Behandlung einer Asthenozoospermie, Oligozoospermie, Teratozoospermie, Oligo-Astheno-Terato-Zoospermie, insbesondere OAT I, OAT II und/oder OAT III, Kryptozoospermie, Parvisemie und/oder zur Verbesserung der Samenqualität und/oder einer beeinträchtigten Kapazitationsfähigkeit.

12. Verwendung nach Anspruch 11, die zumindest ein weiteres Vitamin, insbesondere Ascorbinsäure, umfasst.

13. Verwendung nach Anspruch 11 oder 12, **dadurch gekennzeichnet, dass** das Kombinationspräparat oral in einer Gesamt-Tagesdosis von
- 100 bis 5000 mg, insbesondere ca. 440 mg, L-Carnitin
- 5 bis 250 mg, insbesondere ca. 15 mg, Coenzym Q₁₀
- 50 bis 1000 mg, insbesondere ca. 120 mg, eines Vitamin E, insbesondere α-Tocopherol,
- 5 bis 100 mg, insbesondere ca. 40 mg, einer Quelle für Zink
- 0,1 bis 1 mg, insbesondere ca. 0,8 mg, eines Vitamin B, insbesondere Folsäure,
- 0,01 bis 1 mg, insbesondere ca. 0,06 mg, einer Quelle für Selen
- 10 bis 1000 mg, insbesondere ca. 80 mg, Glutathion
- 100 bis 5000 mg, insbesondere ca. 250 mg, L-Arginin
appliziert wird, wobei vorzugsweise zwei mal täglich jeweils die halbe Dosis appliziert wird.

## Claims

1. Drug combination for improving the semen quality of a male individual comprising as active ingredients in effective quantities:
- L-carnitine,
- coenzyme Q₁₀,
- at least one vitamin E, in particular α-tocopherol,
- at least one source of zinc, in particular zinc sulfate or zinc chloride,
- at least one source of vitamin B, in particular folic acid,
- at least one source of selenium,
- glutathione and
- L-arginine
or salts thereof.

2. Drug combination according to claim 1 comprising:
- 25 to 65 % by weight of L-carnitine, in particular 35 to 60 % by weight, preferably 42 to 55 % by weight,
- 0,5 to 11 % by weight of coenzyme Q₁₀, in particular 1 to 6 % by weight, preferably 1,5 to 4 % by weight,
- 4 to 30 % by weight of avitamin E, in particular α-tocopherol, in particular 7 to 25 % by weight, preferably 10 to 16 % by weight,
- 0,5 to 20 % by weight of a source of zinc, in particular 1 to 15 % by weight, preferably 2 to 7 % by weight,
- 0,01 to 1 % by weight of a vitamin B, in particular of folic acid, in particular 0,05 to 0,3 % by weight, preferably 0,07 to 0,15 % by weight,
- 0,001 to 0,1 % by weight of a source of selenium, in particular 0,002 to 0,03 % by weight, preferably 0,005 to 0,015 % by weight,
- 1 to 25 % by weight of glutathione, in particular 3 to 17 % by weight, preferably 6 to 13 % by weight,
- 15 to 45 % by weight of L-arginine, in particular 20 to 35 % by weight, preferably 22 to 29 % by weight,
or salts thereof, whereby the percentages refer to the total weight of all active ingredients.

3. Drug combination according to claim 1 or 2, **characterized in that** it contains additionally an efficient quantity of lipoic acid, in particular of α-lipoic acid, or salts thereof.

4. Drug combination according to claim 3, **characterized in that** it contains lipoic acid in a quantity of 1 to 20 % by weight, in particular of 3 to 17 % by weight, preferably of 5 to 10 % by weight, with respect to the total weight of all active ingredients.

5. Drug combination according to any of the claims 1 to 4, **characterized in that** it contains additionally an efficient quantity of N-acetylcystein (NAC) or salts thereof.

6. Drug combination according to claim 5, **characterized in that** it contains N-acetylcystein (NAC) in a quantity of 1 to 20 % by weight, in particular of 3 to 17 % by weight, preferably of 5 to 10 % by weight, with respect to the total weight of all active ingredients.

7. Drug combination according to any of the claims 1 to 6, **characterized in that** it contains additionally an efficient quantity of at least one further vitamin, in particular of ascorbic acid, or of salts thereof.

8. Drug combination according to claim 7, **characterized in that** it contains ascorbic acid in a quantity of 4 to 30 % by weight, in particular of 7 to 25 % by weight, preferably of 10 to 16 % by weight, with respect to the total weight of all active ingredients.

9. Drug combination according to any of the claims 1 to 8, **characterized in that** it contains at least one further active ingredient selected from the group consisting of ginseng, vitamin B12 or methylcobalamin, thiamin and/or biotin, in efficient quantities.

10. Drug combination according to any of the claims 1 to 9 consisting in:
- approx. 46 to 47 % by weight of L-carnitine,
- approx. 1,5 to 1,6 % by weight of coenzyme Q₁₀,
- approx. 12 to 13 % by weight of avitamin E, in particular α-tocopherol,
- approx. 4 to 5 % by weight of a source of zinc
- approx. 0,08 to 0,09 % by weight of a vitamin B, in particular folic acid,
- approx. 0,08 to 0,09 % by weight of a source of selenium,
- approx. 8 to 9 % by weight of glutathione
- approx. 26 to 27 % by weight of L-arginine,
whereby the percentages refer to the total weight of all active ingredients.

11. Use of L-carnitine, coenzyme Q₁₀, at least one vitamin E, in particular α-tocopherol, at least one source of zinc, in particular zinc sulfate or zinc chloride,at least one vitamin B, in particular folic acid,at least one source of selenium, glutathione, arginine as well as, if need be, lipoic acid, acetylcystein or other ingredients according to any of the claims 1 to 10 or salts thereof, for producing a drug combination according to any of the claims 1 to 10 for the treatment of reduced fertility or subfertility of a male individual, in particular of a human being, for the treatment or the optimization ofborderline normozoospermia, for the treatment of asthenozoospermia, oligozoospermia, teratozoospermia, oligoasthenoteratozoospermia, in particular OAT I, OAT II and/or OAT III, cryptozoospermia, parvisemia and/or for improving the semen quality and/or an impaired capacitation ability.

12. Use according to claim 11 that comprises at least one further vitamin, in particular ascorbic acid.

13. Use according to claim 11 or 12, **characterized in that** the drug combination is applied orally in a total daily dose of
- 100 to 5000 mg, in particular of approx. 440 mg, of L-carnitine,
- 5 to 250 mg, in particular of approx. 15 mg, of coenzyme Q₁₀,
- 50 to 1000 mg, in particular of approx. 120 mg of a vitamin E, in particular α-tocopherol,
- 5 to 100 mg, in particular of approx. 40 mg, of one source of zinc,
- 0,1 to 1 mg, in particular of approx. 0,8 mg, of a vitaminB, in particular folic acid,
- 0,01 to 1 mg, in particular of 0,06 mg, of a source of selenium,
- 10 to 1000 mg, in particular of approx. 80 mg, of glutathione,
- 100 to 5000 mg, in particular of approx. 250 mg, of L-arginine, whereby preferably half the dose is applied respectively twice daily.

## Revendications

1. Association médicamenteuse pour améliorer la qualité du sperme d'un individu mâle comprenant comme substances actives en quantités efficaces:
- de la L-carnitine,
- la coenzyme Q₁₀,
- au moins une vitamine E, en particulier l'α-tocophérol,
- au moins une source de zinc, en particulier du sulfate de zinc ou du chlorure de zinc,
- au moins une source de vitamine B, en particulier de l'acide folique,
- au moins une source de sélénium,
- du glutathion et
- du L-arginine
ou des sels de celles-ci.

2. Association médicamenteuse selon la revendication 1 comprenant :
- 25 à 65 % en poids, en particulier 35 à 60 % en poids, de préférence 42 à 55 % en poids de L-carnitine,
- 0,5 à 11 % en poids, en particulier 1 à 6 % en poids, de préférence 1,5 à 4 % en poids de coenzyme Q₁₀,
- 4 à 30 % en poids, en particulier 7 à 25 % en poids, de préférence 10 à 16 % en poids d'une vitamine E, en particulier d'α-tocophérol,
- 0,5 à 20 % en poids, en particulier 1 à 15 % en poids, de préférence 2 à 7 % en poids d'une source de zinc,
- 0,01 à 1 % en poids, en particulier 0,05 à 0,3 % en poids, de préférence 0,07 à 0,15 % en poids d'une vitamine B, en particulier d'acide folique,
- 0,01 à 0,1 % en poids, en particulier 0,002 à 0,03 % en poids, de préférence 0,005 à 0,015 % en poids, d'une source de sélénium,
- 1 à 25 % en poids, en particulier 3 à 17 % en poids, de préférence 22 à 29 % en poids de L-arginine
ou des sels de ceux-ci, les indications de pourcentage se rapportant au poids total de toutes les substances actives.

3. Association médicamenteuse selon la revendication 1 ou 2, **caractérisée en ce qu'**en plus elle contient une quantité efficace d'acide liponique, en particulier d'acide α-liponique, ou de sels de celui-ci.

4. Association médicamenteuse selon la revendication 3, **caractérisée en ce que** l'acide liponique est contenu dans une quantité d'1 à 20 % en poids, en particulier de 3 à 17 % en poids, de préférence de 5 à 10 % en poids, par rapport au poids total de toutes les substances actives.

5. Association médicamenteuse selon l'une des revendications 1 à 4, **caractérisée en ce qu'**elle contient de plus une quantité efficace de N-acétylcystéine (NAC) ou de sels de celle-ci.

6. Association médicamenteuse selon la revendication 5, **caractérisée en ce qu'**elle contient de la N-acétylcystéine (NAC) en une quantité d'1 à 20 % en poids, en particulier de 3 à 17 % en poids, de préférence de 5 à 10 % en poids, par rapport au poids total de toutes les substances actives.

7. Association médicamenteuse selon l'une des revendications 1 à 6, **caractérisée en ce qu'**elle contient en plus une quantité efficace d'au moins une autre vitamine, en particulier d'acide ascorbique, ou de sels de celui-ci.

8. Association médicamenteuse selon la revendication 7, **caractérisée en ce qu'**elle contient de l'acide ascorbique en une quantité de 4 à 30 % en poids, en particulier de 7 à 25 % en poids, de préférence de 10 à 16 % en poids, par rapport au poids total de toutes les substances actives.

9. Association médicamenteuse selon l'une des revendications 1 à 8, **caractérisée en ce qu'**elle contient au moins une autre substance active sélectionnée dans le groupe du ginseng, de la vitamine B12 ou de la méthylcobalamine, de la thiamine et/ou de la biotine en quantités efficaces.

10. Association médicamenteuse selon l'une des revendications 1 à 9 constituée par:
- environ 46 à 47 % en poids de L-carnitine,
- environ 1,5 à 1,6 % en poids decoenzyme Q₁₀,
- environ 12 à 13 % en poidsd'une vitamine E, en particulier d'α-tocophérol,
- environ 4 à 5 % en poids d'une source de zinc,
- environ 0,08 à 0,09 % en poids d'une vitamine B, en particulier d'acide folique,
- environ 0,006 à 0,007 % en poids d'une source de sélénium,
- environ 8 à 9 % en poids de glutathion,
- environ 26 à 27 % en poids de L-arginine,
les indications de pourcentage se rapportant au poids total de toutes les substances actives.

11. Utilisation de L-carnitine, decoenzyme Q₁₀, d'au moins une vitamine E, en particulier d'α-tocophérol, d'au moins une source de zinc, en particulier de sulfate de zinc ou de chlorure de zinc, d'au moins une vitamine B, en particulier d'acide folique, d'au moins une source de sélénium, de glutathion, d'arginine ainsi que, le cas échéant, d'acide liponique, d'acétylcystéine ou d'autres substances actives selon l'une des revendications 1 à 10 ou de sels de celles-ci pour le traitement de la fertilité limitée ou de la sous-fertilité d'un individu mâle, en particulier d'un être humain, pour le traitement ou l'optimalisation d'une normozoospermie limite, pour le traitement d'une asthénozoospermie, d'une oligozoospermie, d'une tératozoospermie, d'une oligoasthénotératozoospermie, en particulier d'une OAT I, d'une OTAT II et/ou d'une OTAT III, d'une cryptozoospermie, d'une parvisémie et/ou pour l'amélioration de la qualité du sperme et/ou d'une aptitude limitée à la capacitation.

12. Utilisation selon la revendication 11 qui comprend au moins une autre vitamine, en particulier de l'acide ascorbique.

13. Utilisation selon la revendication 11 ou 12, **caractérisée en ce que** l'association médicamenteuse est appliquée oralement en une dose journalière totale de :
- 100 à 5000 mg, en particulier d'environ 440 mg, de L-carnitine,
- 5 à 250 mg, en particulier d'environ 15 mg, decoenzyme Q₁₀,
- 50 à 1000 mg, en particulier d'environ 120 mg, d'une vitamine E, en particulier d'α-tocophérol,
- 5 à 100 mg, en particulier d'environ 40 mg, d'une source de zinc,
- 0,1 à 1 mg, en particulier d'environ 0,8 mg, d'une vitamine B, en particulier d'acide folique,
- 0,01 à 1 mg, en particulier d'environ 0,06 mg d'une source de sélénium,
- 10 à 1000 mg, en particulier d'environ 80 mg, de glutathion,
- 100 à 5000 mg, en particulier d'environ 250 mg, de L-arginine,
cependant que l'on applique de préférence deux fois par jour respectivement la moitié de la dose.
